## Europäisches Patentamt

## European Patent Office

## Office européen des brevets

(19)

(11) Veröffentlichungsnummer: **0 043 478**
A1

(12) # EUROPÄISCHE PATENTANMELDUNG

(21) Anmeldenummer: **81104738.0**

(22) Anmeldetag: **20.06.81**

(51) Int. Cl.³: **C 07 C 41/42**, C 07 C 41/06

(30) Priorität: **27.06.80 DE 3024147**

(43) Veröffentlichungstag der Anmeldung: **13.01.82**
**Patentblatt 82/2**

(84) Benannte Vertragsstaaten: **AT BE CH DE FR GB IT LI NL SE**

(71) Anmelder: **Edeleanu Gesellschaft mbH,**
**Stresemannallee 36, D-6000 Frankfurt/Main 70 (DE)**

(72) Erfinder: **Prezelj, Milan, Grillparzer Strasse 59,**
**D-6000 Frankfurt/Main (DE)**
Erfinder: **Osterburg, Günter, Buchenstrasse 1,**
**D-4100 Duisburg 17 (DE)**
Erfinder: **Pütz, Joachim, Hunsrückstrasse 14,**
**D-6072 Dreieich-Offenthal (DE)**

(74) Vertreter: **Schupfner, Gerhard, Dr. et al, Müller,**
**Schupfner & Gauger Patentanwälte Karlstrasse 5,**
**D-2110 Buchholz/Nordheide (DE)**

(54) **Verfahren zur Auftrennung der bei der Verätherung von niederen i-Olefinen mit Methanol anfallenden Reaktionsprodukte.**

(57) Verfahren zur Auftrennung der bei der Verätherung von $C_4$–$C_7$ Isoolefinen mit Methanol anfallenden Reaktionsprodukte durch Wasserwäsche in Gegenwart von inerten Kohlenwasserstoffen, wobei der Methanol und tertiäre Alkohole enthaltende Alkoholanteil vollständig mit Wasser extrahiert wird, die wäßrige Extraktlösung sich im Sumpf der Waschkolonne sammelt und von dort in eine Destillationskolonne geleitet wird, in der die Alkohole destillativ abgetrennt werden und tertiärer Alkohol als Seitenstrom abgezogen und in eine Abstreifkolonne übergeführt wird. Das vom Kopf der Abstreifkolonne abgezogene Methanol wird in die Destillationskolonne zurückgeführt, während der im Sumpf der Abstreifkolonne anfallende tertiäre Alkohol ausgeschleust oder dem Strom für die Kohlenwasserstoff/Äther-Trennkolonne zugemischt wird. Der tertiäre Alkohol sammelt sich dann im Sumpf der Trennkolonne als Komponente des trockenen und methanolfreien Ätherproduktes.

0043478

**Verfahren zur Auftrennung der bei der Verätherung von niederen i-Olefinen mit Methanol anfallenden Reaktionsprodukte**

Die Erfindung bezieht sich auf ein Verfahren zur Auftrennung des bei der an sich bekannten Herstellung von unsymmetrischen Äthern durch Umsetzung von $C_4$-$C_7$-iso-Olefinen mit Methanol in Gegenwart eines sauren Katalysators, vorzugsweise eines sauren Kationenaustauscherharzes, erhaltenen Reaktionsgemisches und Abtrennung des Äthers vom nicht umgesetzten Alkohol mittels einer Wasserwäsche mit anschliessender Abscheidung der organischen und wässrigen·Phase.

Vorzugsweise stammt das i-olefinhaltige Einsatzgut der Umsetzung von einem aus der Pyrolyse oder der katalytischen Krackanlage stammenden leichten Kohlenwasserstoff(KW)-Schnitt. Die reagierende Komponenten sind insbesondere i-Buten und i-Penten, der angewendete Alkohol Methanol, die gebildeten Äther insbesondere Methyl-t-butyl-äther (MTBE) und Methyl-t-amyl-äther (TAME), wichtige Zusatzmittel zu Motortreibstoffen.

Das Verfahren zur Herstellung der Äther und die Auftrennung des ätherhaltigen Reaktionsgemisches mittels einer der Reaktorstufe direkt folgenden Waschstufe ist Gegenstand der DE-OS 2547380. Diese Sequenz der Aufbereitungsschritte, nämlich Wäsche vor der destillativen Auftrennung, hat den wesentlichen Vorteil, daß man bei der Methanoldosierung nicht festgelegt ist, und die vollständige Herausnahme des Alkohols in jedem Falle in einem Verfahrensschritt vornimmt, ohne daß man dies mit der Kombination umständlicher Azeotropdestillationen bewerkstelligen muß, wozu ggfl. noch weitere Kolonnen erforderlich sind.

Mit einer geeignet ausgelegten Waschkolonne (5 theoretische Extraktionsstufen, Methanol/Wasser-Verhältnis 1:10 bis 1:20) werden dabei alle Alkohole, also ausser dem Methanol auch die als Nebenprodukte gebildeten tertiären Alkohole, wie t-Butanol (TBA) und t-Amylalkohol (TAA), weitgehend

. . .

aus dem Reaktionsgemisch extrahiert. Die Äther, also MTBE u.a. verbleiben dabei praktisch vollständig in der organischen Phase, da die Anwesenheit der nicht umgesetzten Kohlenwasserstoffe die Ätherverteilung in diesem Sinne begünstigt. Im folgenden wird ausschließlich auf TBA Bezug genommen, obgleich gleiches auch für andere tertiäre Alkohole wie TAA zutrifft.

Der wässrige Extrakt aus dem Sumpf der Waschkolonne enthält ausser Wasser etwa 5-10 % Methanol, 0,5-1 % MTBE und 0,3-0,5 % TBA. Dieses Gemisch wird einer atmosphärischen Destillation unterworfen, um das Methanol zwecks Wiederverwendung zurückzugewinnen.

MTBE geht bei dieser Destillation als Azeotrop mit Methanol über Kopf, wird in den Reaktor zurückgeführt und bereitet in diesem Zusammenhang keine weiteren Schwierigkeiten.

Anders TBA: Bei der Destillation des wässrigen Extraktes destilliert dieser Alkohol als Wasser-Azeotrop und erhöht damit zunächst den Wassergehalt im Methanol-Rückführstrom. Dieses mit dem Rückführstrom in den Reaktor eingeführte Wasser bewirkt seinerseits eine Verschiebung des Reaktionsablaufes der Isobuten-Umsetzung in Richtung der TBA-Bildung. Ein Anreicherungskreislauf mit zusätzlichem "Schneeballeffekt" wäre durch wiederholte Extraktion des TBA in der Wasserwäsche und anschliessende Rückführung zum Reaktor geschlossen, da keine Ausschleusung über die gewaschene organische Phase stattfindet. Eine solche Anreicherung des TBA in dem Methanolkreislauf unterdrückt bereits nach kurzer Betriebszeit die MTBE-Synthese im Reaktor.

Der Erfindung liegt die Aufgabe zugrunde, ein verbessertes Verfahren zur Auftrennung des bei der Synthese von tert. Alkyläthern anfallenden Produktgemisches zu schaffen unter Anwendung einer dem Reaktor direkt nachgeschalteten Wasserwäsche, deren wässriger Extrakt dann destillativ aufgearbeitet wird. Die im wässrigen Extrakt der Waschkolonne

...

0043478

anfallenden Alkoholmengen sollen möglichst vollständig
abgetrennt und wiederverwendet werden.

Diese Aufgabe wird gelöst durch ein Verfahren gemäß Patentanspruch 1, bei dem bei der Destillation des aus der Waschkolonne abgezogenen wässrigen Extraktes das gesamte TBA
mittels eines Seitenabzuges aus der Methanol/Wasser-Kolonne
aus dem Kreislauf entfernt wird. Eine Kontrollvorrichtung
steuert die Menge des Seitenabzugs so, daß am Kopf der Kolonne kein TBA mehr austritt. Dieser Seitenabzug, der der
Wasser/Alkohol-Trennkolonne von einem Boden mit hoher TBA-
Anreicherung entnommen wird, enthält ausser TBA auch noch
beträchtliche Mengen an Methanol (typische Zusammensetzung:
40 % TBA, 50 % Methanol und 10 % Wasser). Diese Methanolmenge kann bis zu 20 % des gesamten aus dem Reaktionsgemisch in der Wäsche zurückgewonnenen Methanols betragen.

Solche Methanolmengen können in einer wirtschaftlich betriebenen Produktionsanlage nicht in Form eines Nebenproduktes verlorengehen. Man kann dieses zwar entweder als
Slop verfeuern oder dem MTBE-Produkt beimischen, doch ist
dieses unwirtschaftlich, bzw. mit einer unerwünschten Verschlechterung der MTBE-Fertigproduktqualität verbunden.

Der TBA-Abzugsstrom aus der Methanol/Wasser-Kolonne wird
daher erfindungsgemäß noch einem kleinen Seitenabstreifer
zugeführt, der den Methanolanteil des TBA-Gemisches als
Dampf wieder der Hauptkolonne zurückführt. Im Sumpf dieses
Seitenabstreifers verbleibt nur noch das wasserhaltige
TBA (ca. 80 % TBA + 20 % Wasser). Dieses Gemisch kann, da
es kein Methanol mehr enthält, dem Strom für die Kohlenwasserstoff/MTBE-Trennkolonne zugemischt werden.
Bei der Destillation in dieser Kolonne geht dann das Wasser
mit den KW-Dämpfen über Kopf und wird im Rücklaufbehälter
abgeschieden. Das TBA wandert in den Sumpf und verlässt den
Prozess als eine Komponente des trockenen und methanolfreien MTBE-Produktes. Auf diese Weise wird eine praktisch

...

quantitative ( > 99.5 %) Ausnutzung des eingesetzten Methanols erreicht und gleichzeitig das Problem der Verwendung des TBA-Nebenproduktes gelöst.

Ebenso wie bei dem beschriebenen einstufigen Verfahren zur Herstellung von MTBE, ist das erfindungsgemäße Verfahren zur Auftrennung des Reaktionsgemisches in einer weiteren Ausführungsform auch bei einem 2-stufigen MTBE-Verfahren anwendbar, wie es beispielsweise aus DE-AS 2706465 bekannt ist.

Bei einem zweistufigen Verfahren wird das den ersten Reaktor verlassende Gemisch einer Destillationskolonne zugeleitet, in der nicht-reagierte Kohlenwasserstoffgemische am Kopf abgezogen werden, damit in einem zweiten Reaktor unter Zuleitung von Frischmethanol die Umsetzung der noch im $C_4$-Kohlenwasserstoffstrom enthaltenen i-Butenrestmenge erfolgt. Entsprechend seiner Zusammensetzung enthält dieser am Kopf der Destillationskolonne abgezogene Kohlenwasserstoff geringe (2-3 %) azeotrope Methanolmengen. Im Sumpf der Kolonne fällt ein Gemisch aus MTBE, TBA und der Hauptmenge des nicht umgesetzten Methanols an. Auch dieses Gemisch wird zum Zweck der Alkohol/Äther-Trennung der mit Wasser arbeitenden Extraktionskolonne zugeführt, nachdem es jedoch vorher mit dem kohlenwasserstoffreichen Produktstrom aus dem zweiten Reaktor vermischt wurde. Die Anwesenheit von Kohlenwasserstoffen bei dem Waschvorgang verhindert bekannterweise den Übergang von MTBE in die wässrige Phase und sorgt für die Gewinnung eines absolut methanolfreien Ätherproduktes, unabhängig davon, welche Methanolmengen den Reaktorstufen zugesetzt wurden.

Wie bei den einstufigen MTBE-Herstellungsverfahren erfolgt erfindungsgemäß die destillative Auftrennung der wässrigen Phase unter Abzug eines Seitenstromes, in den eine kleine Abstreifkolonne eingeschaltet ist. Der methanolhaltige Dampf dieser Seitenkolonne wird wieder in die Methanol-

...

0043478

Destillationskolonne, deren aus TBA bestehendes Sumpfprodukt der Kohlenwasserstoff/MTBE-Trennkolonne zugeleitet.

Auch in diesem Fall der zweistufigen MTBE-Synthese wird
mit den erfindungsgemäßen Maßnahmen eine Ausschleusung der tertiären
Alkohole und eine    restlose Rückführung des nicht umgesetzten Methanols in den Syntheseprozess erreicht.

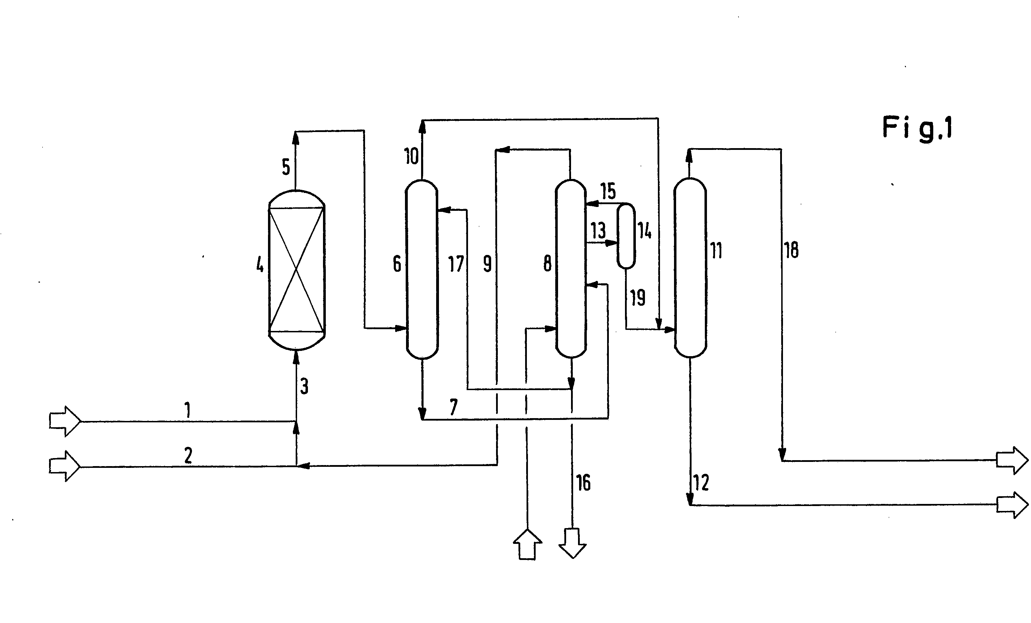

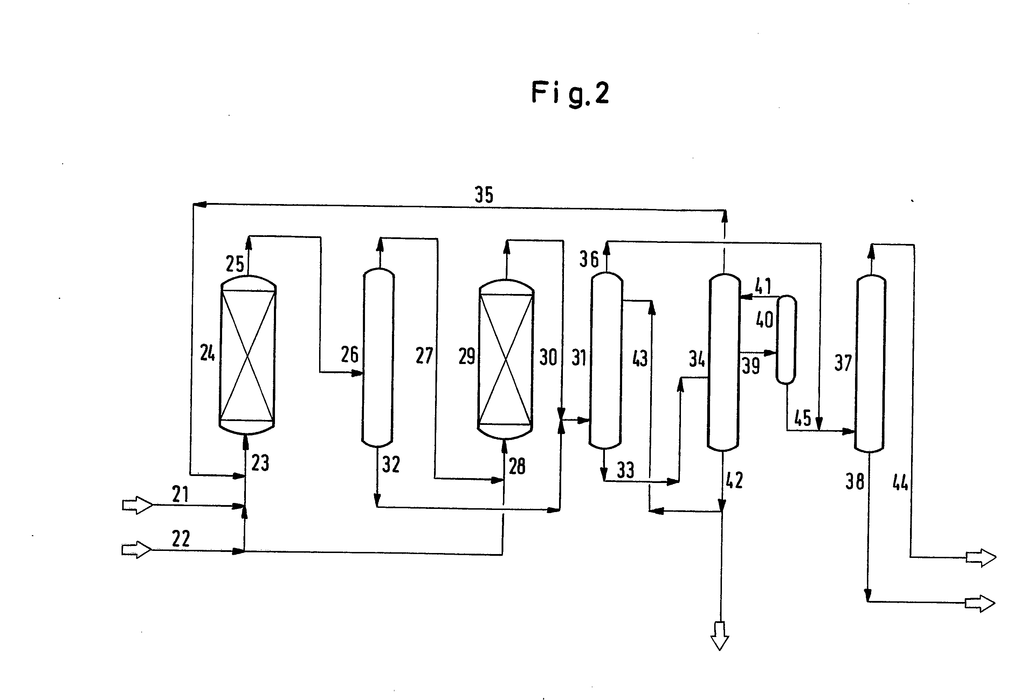

Im folgenden wird das erfindungsgemäße Verfahren, bezogen
auf die Herstellung von MTBE, anhand der Figuren 1 und 2
beschrieben.

<u>Einstufiges Verfahren gemäß Fig. 1</u>

Ein i-butenenthaltender $C_4$-Kohlenwasserstoffstrom aus Leitung 1 und Methanol aus Leitung 2 strömen über Leitung 3
in den Reaktor 4. Das Reaktionsgemisch verlässt über Leitung 5 den Reaktor 4 und wird in den Unterteil der Waschkolonne 6, wo der alkoholische Anteil mit Wasser extrahiert wird, eingeleitet. Im Sumpf der Waschkolonne 6
sammelt sich ein wasserhaltiges Gemisch, daß ausser Wasser etwa 5-10 Gew. % Methanol, 0,5-1 Gew. % MTBE und
0,3-0,5 Gew. % TBA enthält. Dieses Gemisch gelangt über
Leitung 7 in die Destillationskolonne 8, wo das Methanol
über Kopf abgetrieben und über Leitung 9 der dem Reaktor
4 zugehörigen Leitung 3 zugeleitet wird, um wieder an der
Reaktion teilzunehmen. MTBE verlässt gemeinsam mit den
nicht umgesetzten $C_4$-Kohlenwasserstoffen die Waschkolonne
6 über Leitung 10 und strömt durch diese Leitung 10 in
die Destillationskolonne 11, wo das reine MTBE im Sumpf
abgeschieden und durch Leitung 12 ausgetragen wird.
Am Boden der Destillationskolonne 8 mit hoher TBA-Konzentration, einige Böden oberhalb des Eintritts von Leitung
7 in die Kolonne, wird mit Leitung 13 ein TBA-haltiger
Seitenstrom in die kleine Abstreifkolonne 14 geleitet,
deren Größe nur ca 1/10 der Hauptkolonne 8 beträgt.

• • •

Die Menge dieses Seitenstroms ist so bemessen, daß ein Kontrollgerät (nicht gezeigt) am Kopf der Kolonne 8 kein TBA mehr anzeigt. Bezogen auf die in ihm enthaltene Methanolmenge enthält der Seitenstrom etwa 10-20 Gew. % des im wässrigen Extrakt enthaltenen Methanols. Die typische Zusammensetzung dieses Stroms beträgt: etwa 40 Gew. % TBA; etwa 50 Gew. % MeOH und etwa 10 Gew. % $H_2O$. Beheizt wird Kolonne 14 mit einer im Sumpf angeordneten Heizvorrichtung. Der wässrige TBA sammelt sich im Sumpf der Kolonne 14; das Methanol verläßt dampfförmig den Kopf der Kolonne 14 und wird über Leitung 15 wieder der Hauptkolonne 8 zugeleitet. Das im Sumpf der Kolonne 8 anfallende Wasser wird durch Leitung 16 ausgetragen bzw. zum Teil über Leitung 17 in die Waschkolonne 6 überführt. Das den Kopf der Kohlenwasserstoff/MTBE-Trennkolonne 11 verlassende Dampfgemisch enthält nur noch den Anteil der nicht umsetzbaren Komponenten des $C_4$-Kohlenwasserstoffgemisches und verläßt über Leitung 18 die Anlage.

Über Leitung 19 kann der aus dem Sumpf der Kolonne 14 abgezogene wasserhaltige methanolfreie TBA dem Strom in Leitung 10 zugemischt werden und gelangt mit ihm in Kolonne 11. Hier fällt er dann gemeinsam mit dem MTBE als Sumpfprodukt an und wird gemeinsam mit diesem über Leitung 12 ausgetragen, während sein Wassergehalt über Kopf abgeht und nach Kondensation in einem Abscheider (nicht gezeigt) vom $C_4$-Kohlenwasserstoffgemisch abgetrennt wird.

Zweistufiges Verfahren gemäß Fig. 2

Ein i-butenenthaltender $C_4$-Kohlenwasserstoffstrom aus Leitung 21 und Methanol aus Leitung 22 strömen über Leitung 23 in den Reaktor 24. Das Reaktionsgemisch verlässt über Leitung 25 den Reaktor 24 und tritt in die Destillationskolonne 26 ein. Das Kopfprodukt, ein $C_4$-Kohlenwasserstoffgemisch mit einem Restgehalt nicht umgesetzten i-Butens, wird mit Leitung 27 abgezogen und zusammen mit Frisch-

...

methanol aus Leitung 28 in den Reaktor 29 eingeleitet, wo die Umsetzung des restlichen i-Butens erfolgt. Das Reaktionsgemisch, bestehend aus nicht umsetzbaren $C_4$-Kohlenwasserstoffen, Äther und Alkohol geht am Kopf des Reaktors 29 über Leitung 30 ab und betritt die Waschkolonne 31 im unteren Teil derselben, nachdem das über Leitung 32 abgezogene Bodenprodukt der Destillationskolonne 26 zugemischt wurde. In Waschkolonne 31 wird der alkoholische Anteil mit Wasser extrahiert. Im Sumpf der Waschkolonne 31 sammelt sich ein wasserhaltiges Gemisch, das ausser Wasser etwa 5 - 10 Gew. % Methanol, etwa 0,5 - 1 Gew. % MTBE und etwa 0,3 - 0,5 Gew. % TBA enthält. Dieses Gemisch gelangt über Leitung 33 in die Destillationskolonne 34, wo das Methanol über Kopf abgeht und über Leitung 35 der dem Reaktor 24 zugehörigen Leitung 23 zugemischt wird, um wieder an der Reaktion teilzunehmen. MTBE verlässt gemeinsam mit den Kohlenwasserstoff-Komponenten die Waschkolonne 31 am Kopf über Leitung 36 und strömt durch diese Leitung in die Destillationskolonne 37, wo der reine MTBE im Sumpf abgeschieden und durch Leitung 38 ausgetragen wird. Am Boden der Destillationskolonne 34 mit hoher TBA-Konzentration, einige Böden oberhalb des Eintritts von Leitung 33 in die Kolonne, wird mit Leitung 39 ein TBA-haltiger Seitenstrom in die kleine Abstreifkolonne 40 geleitet, deren Größe nur ca 1/10 der Hauptkolonne 34 beträgt. Die Menge dieses Seitenstromes ist so bemessen, daß ein Kontrollgerät (nicht gezeigt) am Kopf der Kolonne 34 kein TBA mehr anzeigt. Bezogen auf die in ihm enthaltene Methanolmenge enthält der Seitenstrom etwa 10-20 Gew. % des im wässrigen Extrakt enthaltenen Methanols. Die typische Zusammensetzung des Seitenstroms beträgt etwa 40 Gew. % TBA; etwa 50 Gew. % MeOH und etwa 10 Gew. % $H_2O$. Beheizt wird diese Kolonne 40 mit einer im Sumpf angeordneten Heizvorrichtung. Der wässrige TBA sammelt sich im Sumpf der Kolonne 40; das Methanol verlässt dampfförmig den Kopf der Kolonne 40 und wird über Leitung 41 wieder der Hauptkolonne 34 zugeleitet. Das im

...

- 9 -

Sumpf der Kolonne 34 anfallende Wasser wird durch Leitung 42
ausgetragen bzw. zum Teil über Leitung 43 in die Waschkolonne
31 überführt.

Das den Kopf der Kolonne 37 verlassende Dampfgemisch enthält
nur noch den Anteil der nicht umsetzbaren Komponenten des $C_4$-
Kohlenwasserstoffgemisches und verläßt über Leitung 44 die Anlage. Über Leitung 45 kann der aus dem Sumpf der Kolonne 40
abgezogene wasserhaltige methanolfreie TBA dem Strom in Leitung 36 zugemischt werden und gelangt mit ihm in Kolonne 37.
Hier fällt er dann gemeinsam mit dem MTBE als Sumpfprodukt
an und wird gemeinsam mit diesem über Leitung 38 ausgetragen,
während sein Wassergehalt über Kopf abgeht und nach Kondensation
mit einem Abscheider (nicht gezeigt) vom $C_4$-Kohlenwasser-
stoffgemisch abgetrennt wird.

Beispiel:

6527 Teile Methanol aus Leitung 2 werden zusammen mit 1679
Teilen Methanol aus Leitung 9 in den Reaktor 4 geführt, wo sie
mit 66488 Teilen des Einsatz-Kohlenwasserstoffes über den aus
Amberlyst 15 bestehenden Katalysator reagieren.

Tabelle I zeigt die Zusammensetzung des KW-Stromes

| Komponente | Mol% | Gew.-Teile |
|---|---|---|
| Isobutan | 31,0 | 21077 |
| n-Butan | 10,1 | 6848 |
| Trans-Buten-2 | 15,6 | 10239 |
| Cis-Buten | 11,2 | 7314 |
| Buten 1 | 13,8 | 9042 |
| Isobuten | 18,3 | 11968 |
| insgesamt | 100,00 | 66488 |

Die Umsetzung im Reaktor erfolgt bei 70°C und 12 bar.

Isobuten und Methanol reagieren unter Bildung eines Reaktionsgemisches, das den gewünschten Methyl-tert. Butyläther enthält.

Der Isobutenumsatz liegt bei 96 %. Das Buten-1, das cis- und trans-Buten-2, das Isobutan und das n-Butan passieren den Reaktor als inerte Kohlenwasserstoffe.
Bei einem Wassergehalt des Einsatzgemisches, bedingt durch im Methanolstrom und KW-Strom mitgeführten geringen Wassermengen von 0,03 %, werden 0,6 % des eingesetzten Isobutens zu TBA umgesetzt.

Der aus dem Reaktor 4 abgezogene Produktstrom hat folgende Zusammensetzung:

Tabelle II

| Komponente | Teile |
|---|---|
| Methanol | 1679 |
| TBA | 90 |
| Isobuten | 478 |
| inerte KW | 54520 |
| Produkt-Äther | 17949 |

In der Waschkolonne 6 wird dieser Produktstrom mit 16000 Teilen Wasser gewaschen (Verhältnis: 90 Teile Wasser, 10 Teile Alkohol). Der Druck in der Waschkolonne beträgt 10 bar, um auch die Kohlenwasserstoffe in flüssiger Phase zu halten. Methanol und TBA werden durch das Wasser restlos extrahiert und sammeln sich als Extrakt im Kolonnensumpf. Der Extrakt enthält
15890 Teile Wasser
1679 Teile Methanol
90 Teile TBA
125 Teile MTBE

Das Raffinat besteht aus:

     17824 Teile MTBE

     54998 Teile inerte Kohlenwasserstoffe

      110 Teile Wasser

und wird im Fraktionierturm 11 in inerte Kohlenwasserstoffe
und MTBE getrennt:

     54998 Teile inerte Kohlenwasserstoffe

     17824 Teile MTBE


Das Wasser wird in einem nicht gezeichneten Abscheider aus
dem Kondensat abgeschieden. Aus der wässrigen Alkoholphase
wird in der mit Strippdampf arbeitenden Methanol-Wassertrennkolonne 8 das Methanol ausgetrieben.
Vom Boden mit der höchsten TBA-Konzentration, dem 28. Boden,
wird ein Seitenstrom abgezogen und einer kleinen Seitenkolonne
14 zugeleitet. Ein Kontrollgerät am Kopf der Hauptkolonne
zeigt hierbei keine wesentlichen TBA-Mengen mehr an.


Es wird ein Seitenstrom abgezogen, der aus

     113 Teilen Methanol

      90 Teilen TBA

      22 Teilen $H_2O$ besteht.


Die 113 Teile Methanol verlassen dampfförmig die Seitenkolonne
14 und gelangen über Leitung 15 wieder in die Hauptkolonne 8,
während 90 Teile TBA und 22 Teile $H_2O$ sich im Sumpf der Seitenkolonne 14 sammeln.


Die Menge an Methanol in dem den Kopf der Kolonne 8 verlassenden Dampf entspricht der in Kolonne 6 ausgewaschenen
Methanolmenge, 1679 Teile. Im Dampf sind weiterhin 125 Teile
gelösten MTBE's enthalten.


Ein im Schema nicht eingezeichneter Methanolrücklaufstrom
hält den Wassergehalt des den Kopf der Kolonne 8 verlassenden
Methanoldampfes unter 0,1 % = unter 1,7 Teilen.

                          ...

0043478

Nach Kondensation wird das Methanol verlustlos in den
Reaktor zurückgeführt.

Das Sumpfprodukt der Seitenkolonne 14 wird zusammen mit
Kopfprodukt der Waschkolonne 6 der Distillationskolonne II
zugeleitet, in der die Kohlenwasserstoffe und das im Sumpfprodukt enthaltene Wasser abgetrennt werden.

Zusammensetzung des Kopfprodukts:

KW      54998 Teile
Wasser    130 Teile

Das Wasser wird nach Kondensation in einem Wasserabscheider
abgeschieden.
Das Bodenprodukt besteht aus

17823 Teilen MTBE
90 Teilen TBA
2 Teilen $H_2O$.

---

Dr. Gerhard S c h u p f n e r
Patentanwalt

D 2110 Buc̵h̵0̵0̵4̵3̵4̵7̵8̵
Karlstr. 5
Tel.: (04181)

den 25.6.80

E - 002 80 DE

## Patentansprüche

1. Verfahren zur Auftrennung der bei der Verätherung von
   $C_4-C_7$ — i-Olefinen mit Methanol in einem Reaktor anfallenden Reaktionsprodukte durch eine dem Reaktor
   direkt nachgeschaltete Wasserwäsche in Gegenwart von
   inerten Kohlenwasserstoffen, wobei der Methanol und tertiäre
   Alkohole enthaltende Alkoholanteil vollständig mit
   Wasser extrahiert wird, die wässrige Extraktlösung sich
   im Sumpf der Waschkolonne sammelt und von da aus in eine
   Destillationskolonne übergeleitet wird, in der die Alkohole und Wasser destillativ getrennt werden, dadurch
   gekennzeichnet, daß aus der Destillationskolonne von
   einem Boden mit hoher Konzentration an tertiärem Alkohol ein
   Seitenstrom abgezogen wird und in eine Abstreiferkolonne
   überführt wird, in der Methanol dampfförmig am Kopf abgezogen und in die Hauptkolonne zurückgeleitet wird,
   während im Sumpf der gesamte tertiäre Alkohol anfällt, der aus
   dem Prozess ausgeschleust oder dem Äther-Produktstrom
   zugemischt wird.

2. Verfahren nach Anspruch 1, dadurch gekennzeichnet,
   daß der Seitenstrom so groß gehalten wird, daß der
   Kopfstrom der Destillationskolonne keinen tertiären Alkohol,
   sondern nur noch Methanol enthält, das dem Prozess
   wieder zugeführt wird.

3. Verfahren nach Anspruch 1 oder 2, dadurch gekennzeichnet,
   daß bei einem zweistufigen Verätherungsprozess, bei dem
   zwischen den beiden Reaktoren eine Destillationsstufe
   zwischengeschaltet ist, das aus Äther, Methanol und tertiärem

...

0043478

Alkohol   bestehende Sumpfprodukt dieser Destillationsstufe mit dem aus dem zweiten Reaktor austretenden,
inerte Kohlenwasserstoffe enthaltenden Reaktionsprodukt vereinigt und mit diesem zusammen in der Waschkolonne mit Wasser gewaschen wird, wobei mit dem anfallenden wässrigen Extrakt ebenfalls nur die alkoholischen Anteile vollständig abgetrennt werden.

Fig.1

# Fig.2

Europäisches
Patentamt

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | <u>US - A - 3 940 450</u> (KUNG-YOU LEE)<br>* Ansprüche 1-5 *<br><br>-- | 1-3 | C 07 C 41/42<br>41/06 |
| | <u>US - A - 4 198 530</u> (W. WENTZ-HEIMER)<br>* Ansprüche 1-3 *<br><br>-- | 1-3 | |
| | <u>US - A - 4 193 770</u> (J.D. CHASE)<br>* Ansprüche 1-6 *<br>& DE - A - 2 854 250<br><br>-- | 1-3 | |
| | <u>DE - A - 2 853 769</u> (HÜLS A.G.)<br>* Seiten 1-8 *<br><br>-- | 1-3 | **RECHERCHIERTE SACHGEBIETE (Int. Cl.³)**<br><br>C 07 C 41/42<br>41/06 |
| P | <u>GB - A - 2 047 706</u> (INSTITUT FRAN-CAIS DU PÉTROLE)<br>* Seiten 1,2 *<br><br>-- | 1-3 | |
| P | <u>GB - A - 2 043 065</u> (INSTITUT FRAN-CAIS DU PÉTROLE)<br>* Seiten 1,2 *<br>& DE - A - 3 015 346<br><br>-- | 1-3 | |
| P | <u>FR - A - 2 449 666</u> (INSTITUT FRAN-CAIS DU PÉTROLE)<br>* Seiten 1-4 *<br>& DE - A - 3 006 104<br><br>-- ./. | 1-3 | |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 12-10-1981 | VERHULST |

EPA form 1503.1 06.78

**EUROPÄISCHER RECHERCHENBERICHT**

Nummer der Anmeldung

| | EINSCHLÄGIGE DOKUMENTE | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| P | <u>GB - A - 2 049 693</u> (INSTITUT FRANCAIS DU PETROLE)<br>  * Seiten 1,2 *<br>& DE - A - 3 017 413<br><br>----- | 1-3 | |
| | | | RECHERCHIERTE SACHGEBIETE (Int. Cl. 3) |